(19) Europäisches Patentamt
European Patent Office
Office européen des brevets

(11) **EP 3 912 555 B1**

(12) **EUROPÄISCHE PATENTSCHRIFT**

(45) Veröffentlichungstag und Bekanntmachung des Hinweises auf die Patenterteilung:
**01.05.2024 Patentblatt 2024/18**

(21) Anmeldenummer: **21164793.8**

(22) Anmeldetag: **25.03.2021**

(51) Internationale Patentklassifikation (IPC):
**A61B 5/11** (2006.01)   **A61F 2/64** (2006.01)
**A61F 5/01** (2006.01)   A61B 5/00 (2006.01)

(52) Gemeinsame Patentklassifikation (CPC):
**A61B 5/1127; A61B 5/1114; A61B 5/112;**
**A61F 2/64; A61F 5/0106; A61F 5/0123;**
A61B 5/6812

(54) **VERFAHREN ZUR BESTIMMUNG DES ROTATIONSPUNKTES EINES KNIEGELENKES, VERFAHREN ZUR HERSTELLUNG EINER KNIEGELENKSENDOPROTHESE ODER EINER KNIEGELENKSÜBERGREIFENDEN ORTHESE UND VORRICHTUNG ZUR DURCHFÜHRUNG EINES VERFAHRENS ZUR BESTIMMUNG DES ROTATIONSPUNKTES EINES KNIEGELENKES**

METHOD FOR DETERMINING THE POINT OF ROTATION OF A KNEE JOINT, METHOD FOR MANUFACTURING A KNEE JOINT ENDOPROSTHESIS OR OVERLAPPING KNEE JOINT ORTHESIS AND A DEVICE FOR THE EXECUTION OF A METHOD FOR DETERMINING THE POINT OF ROTATION OF A KNEE JOINT

PROCÉDÉ DE DÉTERMINATION DU POINT DE ROTATION D'UNE ARTICULATION DU GENOU, PROCÉDÉ DE FABRICATION D'UNE ENDOPROTHÈSE D'ARTICULATION DU GENOU OU D'UNE ORTHÈSE CHEVAUCHANT L'ARTICULATION DU GENOU ET UN DISPOSITIF POUR L'EXÉCUTION D'UN PROCÉDÉ DE DÉTERMINATION DU POINT DE ROTATION D'UNE ARTICULATION DU GENOU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.04.2020 DE 102020204590**

(43) Veröffentlichungstag der Anmeldung:
**24.11.2021 Patentblatt 2021/47**

(73) Patentinhaber: **Pohlig, Kurt**
**83278 Traunstein (DE)**

(72) Erfinder: **Pohlig, Kurt**
**83278 Traunstein (DE)**

(74) Vertreter: **Pfenning, Meinig & Partner mbB**
**Patent- und Rechtsanwälte**
**Theresienhöhe 11a**
**80339 München (DE)**

(56) Entgegenhaltungen:
**WO-A1-2018/213749**

• **Barié Alexander:
"AGA-Komitee-Knie-Ligament",
AGA-Komitee-Knie-Ligament, 31. Dezember 2016 (2016-12-31), Seiten 1-76, XP55853006, Gefunden im Internet:
URL:https://www.aga-online.ch/fileadmin/us er_upload/Komitee_Inhalte/Knie/UH_Knie_Lig ament_Endversion_1_.pdf [gefunden am 2021-10-20]**

**Beschreibung**

Die vorliegende Erfindung betrifft ein Verfahren zur Bestimmung des Rotationspunktes eines Kniegelenkes eines Beines einer Person, ein Verfahren zur Herstellung einer Kniegelenksendoprothese oder einer kniegelenksübergreifenden Orthese und eine Vorrichtung zur Durchführung eines Verfahrens zur Bestimmung des Rotationspunktes eines Kniegelenkes eines Beines einer Person.

Während zur Messung an gesunden Kniegelenken und die Bestimmung von deren Parametern zu nichtdiagnostischen und nichttherapeutischen Zwecken eingesetzt werden können, beispielsweise zur Analyse von Bewegungsmustern und dergleichen, kann die Bestimmung derartiger Parameter an einem Kniegelenk eines Patienten sowohl für nichtdiagnostische und nichttherapeutische Anwendungen als auch für diagnostische und therapeutische Zwecke durchgeführt werden.

Die vorliegende Erfindung betrifft also die oben genannten Verfahren in jedem Sinne, insbesondere jedoch für nichttherapeutische und nichtdiagnostische Zwecke. Das erfindungsgemäße Verfahren kann weiterhin insbesondere bei der Herstellung von Kniegelenksendoprothesen oder -orthesen und/oder der optimierten Implantation von Kniegelenksendoprothesen und/oder bei der individuellen Anpassung von Kniegelenksorthesen eingesetzt werden.

Herkömmliche Kniegelenksendoprothesen und -orthesen sind standardisierte Produkte, die typisiert hergestellt werden. Dabei ist bei der Auswahl der Prothese zur Implantation und/oder bei Implantation selbst die Berücksichtigung verschiedener Parameter des Knies des Patienten wesentlich, insbesondere die Bandspannungen des Innenbandes und/oder des Außenbandes und/oder der Kreuzbänder und/oder insbesondere der Rotationspunkt bzw. die Position der Rotationsachse des Kniegelenks.

Für diese Parameter liegen üblicherweise zum Zeitpunkt der Operation geringe Informationen vor, insbesondere wird weder die Kniegelenksendoprothese selbst noch die Implantation der Kniegelenksendoprothese hinreichend spezifisch auf diese Parameter abgestimmt.

WO 2018/213749 A1 beschreibt ein System und ein Verfahren zur Bestimmung einer Position und Ausrichtung eines Implantats für eine Gelenkersatzoperation. Das System umfasst eine Manschette, die so konfiguriert ist, dass sie von einem Patienten getragen wird und biomechanische Informationen in Bezug auf die Bewegung des Gelenks sammelt. Das System ist so konfiguriert, dass es die gesammelten biomechanischen Informationen empfängt, klinische Messinformationen bezüglich des Gelenks bestimmt, eine muskuloskelettale Simulation für das Gelenk auf der Grundlage der gesammelten biomechanischen Informationen und der bestimmten klinischen Messinformationen erzeugt, einen oder mehrere chirurgische Parameter für den Patienten auf der Grundlage der muskuloskelettalen Simulation bestimmt.

AGA-Komitee-Knie-Ligament (Hrsg.): Themenheft: Diagnostik des Kniebandapparates (Kapitel 3: Stabilitätsprüfung des Kniegelenkes (BARIE, Alexander [et al.]), 2016. URL: https://www.aga-online.ch/komitees/knie-ligament/themenheft-i-diagnostik-deskniebandapparates/) beschreibt verschiedene klinische Tests, die durch einen Arzt durchgeführt werden können um die Stabilität der Bänder des Knies zu bestimmen.

Die vorliegende Erfindung setzt an diesem Stand der Technik ein und stellt Verfahren, insbesondere Verfahren zur Bestimmung von Parametern eines Kniegelenkes gemäß Anspruch 1 und Verfahren zur Herstellung einer Prothese oder Orthese nach Anspruch 8 sowie eine Vorrichtung zur Durchführung der erfindungsgemäßen Verfahren gemäß Anspruch 10 zur Verfügung. Vorteilhafte Weiterbildungen der erfindungsgemäßen Verfahren und der erfindungsgemäßen Vorrichtung werden in den jeweiligen abhängigen Ansprüchen gegeben.

Das erfindungsgemäße Verfahren zur Bestimmung von Parametern eines Kniegelenkes eines Beins einer Person dient insbesondere der Bestimmung der Bandspannung des Innenbandes, der Bandspannung des Außenbandes, der Bandspannung des vorderen und hinteren Kreuzbandes und/oder der Bestimmung des Rotationspunktes des Kniegelenkes einzeln oder in Kombination. Die folgenden Ausführungen gelten nicht nur für Kniegelenksendoprothesen, sondern - auch wenn nicht extra erwähnt - für kniegelenksübergreifende Orthesen.

Bei dem erfindungsgemäßen Verfahren wird das Kniegelenk der Person einer, mehreren oder sämtlichen der folgenden forcierten Bewegungen bzw. Kräften statisch und/oder dynamisch ausgesetzt:

Va lgusstress,
Varusstress,
Flexion,
Extension und/oder
sagittale Translation.

Während der forcierten Bewegung wird gleichzeitig die relative Position des Unterschenkels und des Oberschenkels des Beins bestimmt. Aus diesen Positionsdaten lässt sich dann einer oder mehrere der obigen Parameter des jeweiligen Kniegelenks bestimmen. Vorteilhaft ist es hierbei, wenn bei der Bestimmung von Parametern mehrerer Kniegelenke die forcierten Bewegungen und Kräfte bei allen Kniegelenken mit gleicher Auslenkung und/oder gleicher Kraft erfolgen.

Auf diese Weise lässt sich für ein individuelles Kniegelenk sowohl die Bandspannung des Innenbandes, des Außenbandes und/oder der Kreuzbänder als auch der Rotationspunkt des Kniegelenks bestimmen. Besonders vorteilhaft ist es, wenn das Verfahren auf eine Vielzahl von Personen bzw. Kniegelenken angewandt wird und auf diese Weise statistische Daten über die oben genannten Parameter der Bandspannungen und des Rotationspunktes des Kniegelenks erhalten werden.

Die erfindungsgemäßen Verfahren werden insbesondere an gesunden Personen durchgeführt, so dass sie nicht zu therapeutischen Zwecken eingesetzt werden. Dennoch lassen sich hierdurch statistische Werte für die genannten Parameter ermitteln, die anschließend beispielsweise bei der Produktion typisierter bzw. standardisierter Kniegelenksendoprothesen verwendet werden können.

In diesem Fall wird folglich das erfindungsgemäße Verfahren weder zu diagnostischen Zwecken (von einzelnen Personen individuell) noch zu therapeutischen Zwecken (an erkrankten Personen) eingesetzt, sondern lediglich zur Herstellung von möglichst weitgehend den Verhältnissen in gesunden Knien entsprechenden Kniegelenksendoprothesen oder -orthesen.

Das Verfahren lässt sich jedoch auch individualisiert an Patienten einsetzen, beispielsweise indem nach Erhebung der Parameter des Knies eines Patienten eine Endoprothese gemäß der für diesen Patienten zu einem früheren Zeitpunkt erhobenen Parameter implantiert wird. Eine derartige individualisierte Medizin mit Bestimmung der o.g. Parameter am noch gesunden Kniegelenk zur späteren Anwendung bei der Implantation einer Kniegelenksendoprothesen ist heutzutage noch nicht üblich.

Auch in letzterem Fall werden insbesondere die Parameter des Kniegelenks des Patienten möglichst bereits am gesunden Kniegelenk des Patienten, d. h. vor Auftreten der Erkrankung, erhoben. Es handelt sich also auch hier nicht um eine diagnostische Verwendung zur Diagnose einer Erkrankung. Dennoch lässt sich aufgrund dieser Daten dann anschließend, im Erkrankungsfall, eine Kniegelenksendoprothese herstellen, die physiologisch weitgehend dem gesunden Kniegelenk des Patienten entspricht und/oder gemäß der erhobenen Parameter individualisiert implantieren, so dass die physiologische Situation der Kniegelenksendoprothese weitgehend derjenigen des gesunden Knies des Patienten entspricht.

Soweit zulässig, schließt die vorliegende Erfindung die diagnostische und/oder therapeutische Verwendung des erfindungsgemäßen Verfahrens jedoch ein, bzw. im gegenteiligen Fall aus.

Das erfindungsgemäße Verfahren kann nicht nur am gesunden Knie und/oder am kranken Knie, sondern auch nachträglich an einem bereits mit einer Endoprothese versehenen Knie durchgeführt werden. Dies ermöglicht die Kontrolle des durch die Endoprothese erzielten therapeutischen Ergebnisses. Eine derartige Nachkontrolle des therapeutischen Ergebnisses kann dann in die Planung typisierter und standardisierter Kniegelenksendoprothesen einfließen. Auch in diesem Fall kann die Erhebung der oben genannten Parameter durch das erfindungsgemäße Verfahren sowohl in diagnostischer und therapeutischer Anwendung an einem einzelnen Patienten erfolgen, als auch in nichtdiagnostischer und nichttherapeutischer Verwendung, soweit beispielsweise die so erhobenen Parameter nicht in die weitere Behandlung des Patienten einfließen, sondern in die Verbesserung bei der Herstellung weiterer, nicht für diesen Patienten individualisierter, sondern standardisierter und/oder typisierter Kniegelenksendoprothesen oder Kniegelenksorthesen.

Die oben genannten forcierten Bewegungen können durch eine dritte Person, eine Vorrichtung oder auch durch eine willkürliche Bewegung der Person selbst, insbesondere beispielsweise während des Gangs der Person, bestimmt werden. Das Verfahren kann also der Erfassung der individuellen Kniegelenkskinematik eines Menschen dienen, der sich nicht im erkrankten Zustand befindet, d. h. insbesondere bei normalem Gang.

Insbesondere eignet sich für die Durchführung des Verfahrens die Vorrichtung nach Anspruch 10. Diese weist Positionsbestimmungsvorrichtungen zur Bestimmung der relativen Position zwischen Unterschenkel und Oberschenkel des Beins auf. Wenn das Kniegelenk einer oder mehreren oder sämtlichen der folgenden forcierten Bewegungen:

Valgusstress,
Varusstress,
Flexion,
Extension und/oder
sagittale Translation

ausgesetzt wird, können die oben genannten Parameter aus der Relativposition des Unterschenkels und des Oberschenkels bestimmt werden. Die Erzeugung der forcierten Bewegungen kann dabei mechanisch durch die Vorrichtung oder durch eine dritte Person erfolgen. Die Erzeugung der forcierten Bewegungen durch eine Vorrichtung hat den Vorteil, dass definierte Kräfte oder Wege angewandt werden können und so die erhobenen Parameter standardisiert erhoben werden. Ihre Vergleichbarkeit, statistische Aussagekraft und Reproduzierbarkeit werden dadurch verbessert.

Zur Bestimmung der relativen Position des Unterschenkels und des Oberschenkels kann sowohl unmittelbar die relative Position zueinander oder gegenüber einer weiteren Position bestimmt werden, als auch die jeweilige absolute Position von Unterschenkel und Oberschenkel in einem vorgegebenen Koordinatensystem ermittelt werden, wobei dann während der forcierten Bewegung, bzw. vor und am Ende der forcierten Bewegung, die relative Position von Oberschenkel und Unterschenkel aus diesen absoluten Koordinaten ermittelt wird.

Die Positionsbestimmung kann beispielsweise erfolgen, indem am Bein, insbesondere am Unterschenkel und am Oberschenkel, Positionsmarker angebracht werden und während der forcierten Bewegung das Bein, insbesondere Unter- und Oberschenkel, mit einer oder mehreren Kameras erfasst werden. Besonders vorteilhaft ist ein 3D-Scan des Unterschenkels und des Oberschenkels während der forcierten Bewegung.

Alternativ ist es auch möglich, unmittelbar am Unterschenkel und Oberschenkel oder an geeigneten anderen Stellen Positionssensoren oder Bewegungssensoren anzubringen. Diese erfassen unmittelbar die jeweilige Position bzw. Bewegung des Beins während der forcierten Bewegung und liefern so unmittelbar (Positionssensoren) oder nach Weiterverarbeitung der erhobenen Daten (Bewegungssensoren) die relative Position zwischen Unter- und Oberschenkel. Als Positions- und/oder Bewegungssensor eignen sich insbesondere Gyroskope, wobei am Unter- und am Oberschenkel vorteilhafterweise jeweils mindestens ein Gyroskop angebracht wird.

Es ist neben der Positionierung am Unterschenkel und am Oberschenkel von jeweils mindestens einem Positions- und/oder Bewegungssensor und/oder Marker auch möglich, zwei oder mehr Sensoren und/oder Marker am Unterschenkel und/oder ebenso am Oberschenkel einzusetzen oder diese an anderen Positionen des Beins anzubringen.

Es ist auch möglich, die Position des Beins, insbesondere die relative Position zwischen Unterschenkel und Oberschenkel des Beins, unmittelbar über einen 3D-Scan zu erfassen, so dass keinerlei Marker oder Sensoren an dem Bein angebracht werden müssen.

Sämtliche vorstehenden Ausführungen gelten für kniegelenksübergreifende Orthesen in gleicher Weise.

Die vorliegende Erfindung betrifft auch ein Verfahren zur Herstellung einer Kniegelenksendoprothese oder einer kniegelenksübergreifenden Orthese, in die die Parameter des Kniegelenks oder des Gelenks, für welches die Orthese gedacht ist, einfließen und die nach dem vorbeschriebenen Verfahren zur Erfassung von Parametern bestimmt werden. Besonders relevant ist dabei nicht etwa die Erfassung der Parameter einer einzelnen Person und Herstellung einer Kniegelenksendoprothese oder einer kniegelenksübergreifenden Orthese anhand der so bestimmten individuellen Parameter, sondern insbesondere die Erfassung von Parametern von Kniegelenken oder anderer Gelenke einer Vielzahl von Personen zur Herstellung standardisierter Prothesen oder Orthesen. Denn die Daten über die Eigenschaften von Kniegelenken einer Mehrzahl von Personen können dann unter Verwendung statistischer Verfahren ausgewertet werden und anschließend in die Herstellung von Kniegelenksendoprothesen und/oder kniegelenksübergreifenden Orthesen, die typisiert oder standardisiert sind, eingebracht werden. Damit ist es mögliche, eine Anzahl unterschiedlicher Typen von Prothesen und/oder Orthesen herzustellen, aus denen der Therapeut eine, für einen individuellen Patienten, geeignete Prothese und/oder Orthese auswählen kann.

Die Erfindung betrifft weiterhin ein Verfahren zur intraoperativen individuellen Anpassung der Spannung eines oder mehrerer der Seitenbänder und/oder Kreuzbänder eines Kniegelenks einer Person. Liegen die oben beschriebenen Parameter für das therapierte Kniegelenk des Patienten bei gesundem Kniegelenk des Patienten vor, so ist es möglich, anhand dieser Parameter das Kniegelenk bzw. die entsprechenden Bandspannungen intraoperativ dem gesunden Zustand der Person anzupassen.

Beispielsweise kann das erfindungsgemäße Verfahren auch bei Kreuzbandrupturen oder sonstigen ligamentären Erkrankungen oder Verletzungen, welche die Kniegelenkskinematik beeinflussen, therapeutisch und nicht-therapeutisch genutzt werden. Es können, zum Beispiel, präoperative die Parameter des erkrankten und des kontralateralen, gesunden Knies erhoben werden um als Orientierung für die Operation zu dienen. Postoperative, beispielsweise nach einer Kreuzbandersatzoperation, kann die Erhebung der Parameter durch das erfindungsgemäße Verfahren und deren Vergleich mit den präoperativ erhobenen Parametern zur Verlauf- bzw. Erfolgskontrolle der Behandlung verwendet werden.

Es soll hier noch einmal betont werden, dass nicht nur die entsprechenden Parameter für die Bandspannung durch die erfindungsgemäßen Verfahren und Vorrichtungen erfasst werden, sondern es auch möglich ist, den individuellen Rotationspunkt eines Kniegelenks eines Patienten zu bestimmen. Dieser spielt eine große Rolle für die spätere Verschleißfestigkeit und Bewegungsabläufe der Endoprothese bzw. für die korrekte Ausrichtung der Orthese. Insbesondere ist es möglich, eine Vielzahl von Daten über Rotationspunkte von Kniegelenken zu erfassen und auf diese Weise die Herstellung standardisierter und/oder typisierter Kniegelenksendoprothesen oder -orthesen zu verbessern.

Im Folgenden werden einige Beispiele erfindungsgemäßer Verfahren und Vorrichtungen gegeben. Dabei bezeichnen gleiche und ähnliche Bezugszeichen gleiche und ähnliche Elemente, so dass deren Beschreibung gegebenenfalls nicht wiederholt wird.

In den folgenden Beispielen wird eine Vielzahl von zwingenden und optionalen Merkmalen jeweils in Kombination beschrieben. Es ist dennoch möglich, auch eine Kombination von einzelnen Merkmalen aus verschiedenen Beispielen zu realisieren bzw. optionale Merkmale verschiedener Beispiele miteinander gemeinsam zu realisieren, ohne jeweils sämtliche optionalen Merkmale der jeweiligen Beispiele zu realisieren.

Es zeigen

Figur 1 ein erfindungsgemäßes Verfahren zur Bestimmung einer Seitenbandspannung (Bandspannung von Innen- und Außenband) eines Knies;

Figur 2 ein erfindungsgemäßes Verfahren zur Bestimmung einer Spannung des Kreuzbandes eines Knies;

Figur 3 ein erfindungsgemäßes Verfahren zur Bestimmung der Position der Rotationsachse RA eines Knies; und

Figur 4 eine Darstellung des verwendeten Ellipsenmodells des Verfahrens der Figur 3.

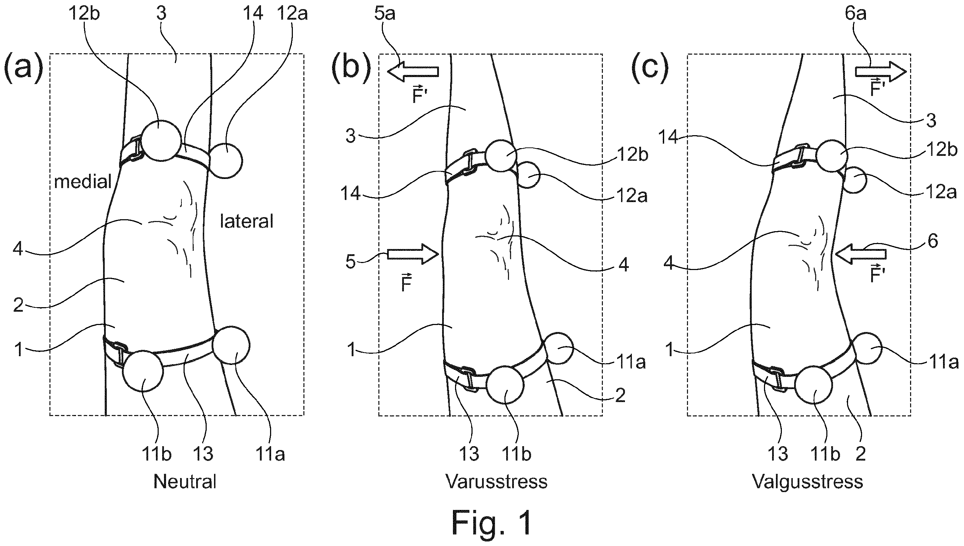

In den Figuren 1 (a)-(c) werden die verschiedenen Schritte zur Bestimmung der Seitenbandspannung des Kniegelenks 4 eines Patienten gezeigt. Die Bestimmung erfolgt in unterschiedlichen Flexionsgraden des Kniegelenks. Fig. 1 (a)-(c) zeigen exemplarisch die Bestimmung in Extension.

In Figur 1 (a) ist ein Bein 1 mit einem Oberschenkel 2 und einem Unterschenkel 3 sowie einem Kniegelenk 4 in neutraler Position, d. h. in von außen unbelastetem Zustand, dargestellt. Auf der linken Bildseite ist die mediale Seite des Beins 1 und auf der rechten Bildseite die laterale Seite des Beins 1 definiert. Das Bein 1 ist am Oberschenkel 2 mit zwei Markern 11a und 11b versehen, wobei die beiden Marker 11a und 11b mittels eines Haltebandes 13 miteinander verbunden und am Oberschenkel 2 befestigt sind. In gleicher Weise ist der Unterschenkel 3 mit Markern 12a und 12b versehen, die mittels eines Haltebands 14 miteinander verbunden und am Unterschenkel 3 befestigt sind. Als Marker 11a, 11b, 12a, 12b werden hier Markerkugeln verwendet um die Position von Ober- und Unterschenkel 2, 3 und damit auch die Relativposition von Ober- und Unterschenkel 2, 3 zueinander zu erfassen. Die Messung der Positionen der Marker 11a, 11b, 12a, 12b kann mittels Foto- und/oder Videoaufnahmen erfolgen. Alternativ oder zusätzlich zu den Markern 11a, 11b, 12a, 12b können andere Marker oder auch Sensoren am Oberschenkel 2 und/oder Unterschenkel 3 angeordnet werden. Hierfür eignen sich beispielsweise Gyroskope, mit denen während der Erzeugung einer forcierten Bewegung die Bewegung und/oder Position von Ober- und Unterschenkel 2, 3 erfasst werden.

Für das erfindungsgemäße Verfahren werden nun nacheinander eine oder mehrere Zwangsbewegungen an dem Kniegelenk 4 bzw. Bein 1 einer Person durchgeführt. In Fig. 1 (b) wird, um das Kniegelenk 4 einem Varusstress auszusetzen, dieses von der medialen Seite mit einer Kraft 5 in lateraler Richtung belastet und gleichzeitig der Unterschenkel 3 durch beispielsweise Aufwendung einer gleichen Gegenkraft 5a in mediale Richtung fixiert. Die durch die Kraft erzeugte forcierte Bewegung kann durch einen Dritten, durch eine Vorrichtung oder durch eine willkürliche Bewegung der Person, insbesondere durch den Gang der Person, erzeugt werden. In der Fig. 1 (b) wird die Kraft 5 von einem Dritten in laterale Richtung auf das Kniegelenk 4 aufgewendet. Währenddessen wird die Position der Marker 11a, 11b, 12a, 12b vor der Anwendung der Kraft 5 sowie während der Anwendung der Kraft 5 gemessen. Die Messung der Positionen der Markern 11a, 11b, 12a, 12b erfolgt mittels Foto- und/oder Videoaufnahmen. Die Bandspannung des Außenbandes kann aus der Differenz der Distanz der seitlichen Marker 11a, 12a in der neutralen Position des Kniegelenks 4 und der Distanz der seitlichen Marker 11a, 12a, wenn das Kniegelenk 4 unter Varusstress (Dehnung des Außenbandes) gesetzt wird, bestimmt werden. Dieselben Messungen können ebenfalls anhand der Marker 11b, 12b durchgeführt werden. Welche Marker 11a, 11b, 12a, 12b besser geeignet sind hängt von deren Positionierung am Oberschenkel 2 bzw. Unterschenkel 3, sowie der Aufnahmerichtung der Foto- und/oder Videoaufnahme ab.

Die Messungen in neutraler Position des Kniegelenks 4 und wenn das Kniegelenk 4 unter Varusstress steht werden in voller Extension des Kniegelenks 4, wie in Fig. 1 (a) und Fig. 1 (b) dargestellt, durchgeführt und anschließend bei verschiedenen Flexionsgraden des Kniegelenks 4 (z. B. Flexion von 30°, 60°, 90°) wiederholt. Die Zwischenwinkel der Flexion sind für die Messung vorteilhaft, da die meisten Kniegelenke 4 im Verlauf des Bewegungsumfangs von Extension (0°) zu einer Flexion von 90° eine sich verändernde Bandspannung aufweisen. Des Weiteren dient bei voller Extension die Kniegelenkskapsel als starker Stabilisator und kann dadurch eine hohe Bandspannung suggerieren. Je weiter fortgeschritten die Flexion des Kniegelenks 4 ist, desto geringer wird der Einfluss der dorsalen Kniegelenkskapsel als Stabilisator und die Bandspannung der Seitenbänder kann erfasst werden. Weiterhin kommt es aus physiologischen Gründen zu unterschiedlichen Bandspannungen des Innen- und Außenbandes beim Übergang aus der Extension in die Flexion. So sind die Bandspannungen beider Bänder in der Extension weitgehend ausgeglichen, in der Beugung (Flexion um 90°) weist das Außenband dagegen in vielen Fällen eine geringere Spannung als das Innenband auf. Das Zusammenspiel all dieser Faktoren macht eine Messung bei verschiedenen Flexionsgraden (bspw. 0°, 30°, 60° und 90°) vorteilhaft.

Zur Ermittlung der Bandspannung des Bandes auf der medialen Seite (Innenband) wird das Kniegelenk 4 einer Person einem Valgusstress (Dehnung des Innenbandes), wie in Fig. 1 (c) gezeigt, ausgesetzt. Gleiche Bezugszeichen verweisen dabei auf gleiche Merkmale wie in Fig. 1 (a) und Fig. 1 (b) und werden nicht nochmals gesondert erläutert. Dabei wird das Kniegelenk 4 von der lateralen Seite mit einer Kraft 6 in mediale Richtung belastet und gleichzeitig der Unterschenkel 3 durch beispielsweise Aufwendung einer gleichen Gegenkraft 6a fixiert in laterale Richtung. Die durch die Kraft erzeugte forcierte Bewegung des Knies kann wiederum durch einen Dritten (wie in Fig. 1 (c) gezeigt), durch eine Vorrichtung oder durch eine willkürliche Bewegung der Person, insbesondere durch den Gang der Person, erzeugt werden. Zur Bestimmung der Bandspannung des Innenbandes werden wiederum die Positionen der Marker 11a, 11b, 12a, 12b in der neutralen Position (Fig. 1 (a)) des Kniegelenks 4 und des Kniegelenks 4 unter Valgusstress (Fig. 1 (c)) gemessen. Die Messung der Positionen der Marker 11a, 11b, 12a, 12b kann, wie zuvor beschrieben, mittels Foto- und/oder Videoaufnahmen erfolgen. Die Bandspannung des Innenbandes kann dann, ähnlich zum Fall der Bandspannung des Außenbandes, aus der Differenz der Distanz der seitlichen Marker 11a, 12a in der neutralen Position des Kniegelenks 4 und der Distanz der seitlichen Marker 11a, 12a, wenn das Kniegelenk 4 unter Valgusstress gesetzt wird, bestimmt werden. Hierbei lassen sich wiederum auch die Marker 11b, 12b verwenden, wobei, wie bereits bei den Messungen unter Varusstress beschrieben, die Positionierung der Marker 11a, 11b, 12a, 12b, sowie die Richtung der Foto- und/oder Videoaufnahme eine ausschlaggebende Rolle spielen, welche Marker für die Messungen besser geeignet sind.

Die Messungen in neutraler Position des Kniegelenks 4 und wenn das Kniegelenk 4 unter Valgusstress steht werden in voller Extension des Kniegelenks 4, wie in Fig. 1 (a) und Fig. 1 (c) zu sehen, durchgeführt und anschließend bei verschiedenen Flexionsgraden des Kniegelenks 4 (z. B. Flexion von 30°, 60°, 90°) wiederholt. Die verschiedenen Flexionsgrade sind hierbei aus denselben Gründen notwendig, die schon bei der Beschreibung der Messungen unter Varusstress beschrieben wurden.

Alternativ kann die Positionserfassung für Oberschenkel 2, Unterschenkel 3 und/oder Kniegelenk 4 des Beins 1 auch mittels Bewegungssensoren (z. B. Gyroskopen) durchgeführt werden.

Aus den so bestimmten Daten lassen sich dann die Bandspannungen der Seitenbänder und/oder Kreuzbänder bestimmen. Durch Erhebung einer Vielzahl derartiger Daten an einer Vielzahl von Personen ist es möglich, typisierte Parameter für Bänder eines gesunden Kniegelenkes 4 zu erzeugen. Es ist auch möglich, typisierte Parameter für bestimmte Personengruppen, beispielsweise gegliedert nach Alter, Körpergröße, sportlicher Belastung etc. zu erfassen. Es ist dadurch möglich, typisierte und/oder standardisierte Kniegelenksendopro-thesen oder auch typisierte und/oder standardisierte Kniegelenksorthesen herzustellen.

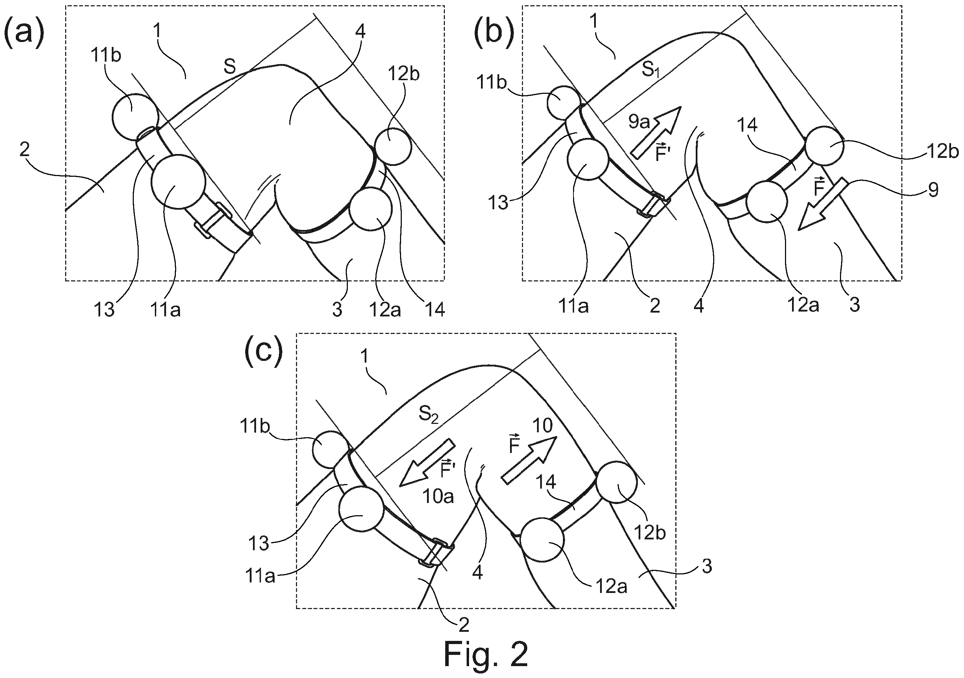

Fig. 2 (a)-(c) zeigen verschiedene Positionen eines Beins 1, die zur Bestimmung der Spannung des hinteren und vorderen Kreuzbandes des Kniegelenks 4 eines Beins1 verwendet werden. Die verwendeten Bezugszeichen verweisen dabei auf entsprechende Merkmale wie in Fig. 1.

Für das erfindungsgemäße Verfahren zur Bestimmung der Bandspannung bzw. -stabilität des Kreuzbandes eines Kniegelenks 4 werden die Messungen, wie in Fig. 2 (a) dargestellt, im Sitzen bei einer Flexion des Kniegelenks 4 von 90° durchgeführt. Das Bein 1 ist dabei mit denselben Markern 11a, 11b, 12a, 12b wie in Fig. 1 versehen, wobei sich die Marker 11a, 11b, 12a, 12b ebenfalls an denselben Positionen wie in Fig. 1 befinden. Zuerst wird, wie in Fig. 2 (a) dargestellt, die Strecke S, welche als Abstand der ventralen Enden der Markern 11b und 12b definiert ist, ohne Aufwendung einer Kraft auf das Kniegelenk 4 gemessen. Wie bereits bei der Bestimmung der Bandspannung des Innen- und Außenbandes des Kniegelenks 4, kann die Messung der Positionen der Marker 11b, 12b durch Foto- und/oder Videoaufnahmen erfolgen. Alternativ oder zusätzlich zu den Markern 11a, 11b, 12a, 12b können andere Marker oder auch Sensoren am Oberschenkel 2 und/oder Unterschenkel 3 angeordnet werden. Hierfür eignen sich beispielsweise Gyroskope, mit denen während der Erzeugung einer forcierten Bewegung die Bewegung und/oder Position von Ober- und Unterschenkel 2, 3 erfasst werden.

Zur Bestimmung der Bandspannung bzw. -stabilität der Kreuzbänder des Kniegelenks 4 wird das Kniegelenk 4 einer sagittalen Translation in verschiedene Richtungen ausgesetzt. Dabei ist zu beachten, dass die Bandspannungen der beiden Kreuzbänder nicht unabhängig voneinander bestimmt werden können, allerdings je nach Richtung der sagittalen Translation die Spannung des einen Kreuzbandes dominiert, während die Spannung des anderen Kreuzbandes eine untergeordnete Rolle spielt.

Fig. 2 (b) zeigt die Erzeugung einer sagittalen Translation durch Aufwendung einer Kraft 9 auf das Schienbein in Richtung der Wade. Die dazugehörige Gegenkraft 9a wird durch die sitzende Position und die damit verbunden Fixierung des Oberschenkels 3 erzeugt. Die durch die Kraft erzeugte forcierte Bewegung kann durch einen Dritten, wie in Fig. 2 (b) dargestellt, oder durch eine Vorrichtung erzeugt werden. Zur Bestimmung der Spannung bzw. Stabilität des hinteren Kreuzbandes wird dabei die Strecke $S_1$ gemessen, die entsprechend der Strecke S als Abstand der ventralen Enden der Marker 11b, 12b definiert ist. Die Spannung bzw. Stabilität des hinteren Kreuzbandes lässt sich mithilfe der Differenz $S-S_1$ bestimmen. Dabei ist zu beachten, dass, wie bereits oben erwähnt, die Bestimmung der Spannung bzw. Stabilität des hinteren Kreuzbandes nicht vollkommen unabhängig vom vorderen Kreuzband erfolgt, dieses aber bei der Messung nur eine untergeordnete Rolle spielt.

Fig. 2 (c) zeigt die Erzeugung einer sagittalen Translation durch Aufwendung einer Kraft 10 auf die Wade in Richtung des Schienbeins. Die dazugehörige Gegenkraft 10a wird durch die sitzende Position und die damit verbunden Fixierung des Oberschenkels 3 erzeugt. Die durch die Kraft erzeugte forcierte Bewegung kann durch einen Dritten, wie in Fig. 2 (c) dargestellt, oder durch eine Vorrichtung erzeugt werden. Zur Bestimmung der Spannung bzw. Stabilität des vorderen Kreuzbandes wird dabei die Strecke $S_2$ gemessen, die entsprechend der Strecke S als Abstand der ventralen Enden der Marker 11b, 12b definiert ist. Die Spannung bzw. Stabilität des vorderen Kreuzbandes lässt sich mithilfe der Differenz $S_2-S$ bestimmen. Es ist zu beachten, dass, wie bereits oben erwähnt, die Bestimmung der Spannung bzw.

Stabilität des vorderen Kreuzbandes nicht vollkommen unabhängig vom hinteren Kreuzband erfolgt, dieses aber bei der Messung nur eine untergeordnete Rolle spielt.

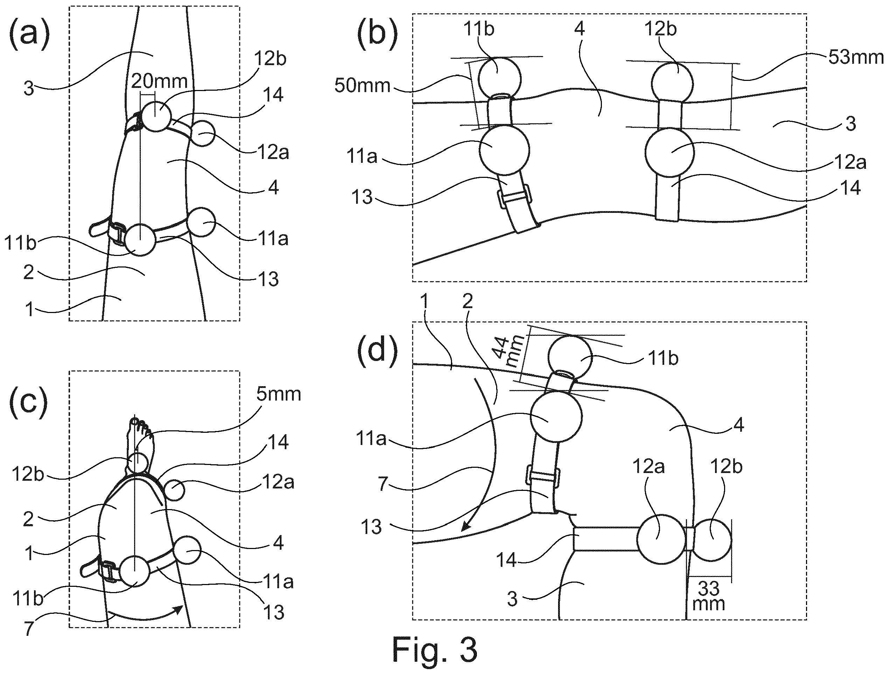

Fig. 3 (a)-(d) zeigen verschiedene Positionen eines Beins 1, die zur Bestimmung der Lage der horizontalen Rotationsachse RA eines Kniegelenks 4 des Beins 1 verwendet werden. Die verwendeten Bezugzeichen verweisen dabei auf entsprechende Merkmale wie in Fig. 1.

In Fig. 3 (a) ist das Kniegelenk 4, wie in Fig. 1, in Draufsicht in einem Normalzustand gezeigt. Das Bein 1 befindet sich in Extension. Am Oberschenkel 2 des Beins 1 sind Marker 11a, 11b und am Unterschenkel 3 des Beins 1 sind Marker 12a, 12b mithilfe von Haltebändern 13, 14 befestigt. Fig. 3 (b) zeigt eine Seitenansicht aus lateraler Richtung desselben Kniegelenks 4 in derselben Stellung wie in Fig. 1 (a). Weiterhin sind in Fig. 3 (a) und Fig. 3 (b) exemplarische Werte für die relativen Positionen der Marker 11a, 11b, 12a und 12b zueinander gezeigt. Der laterale Abstand der Mittelpunkte der Marker 11b und 12b beträgt 20 mm. Der Abstand der Enden in ventraler Richtung der Marker 11a und 11b sowie der Marker 12a und 12b beträgt in Seitenansicht 50 mm bzw. 53 mm.

Die Fig. 3 (c) und Fig. 3 (d) zeigen dieselben Ansichten wie Fig. 3 (a) und Fig. 3 (b) mit dem Unterschied, dass sich das Kniegelenk 4 in Flexion mit einem Winkel von 90° befindet. Der Richtungspfeil 7 zeigt in Fig. 3 (c) und Fig. 3 (d) die Rotationsrichtung des Femurkondylen (Oberschenkelknochenfortsatz mit Gelenkknorpel, der ein Teil des Kniegelenks 4 ist) bei zunehmender Flexion (aus der Extension kommend) an. Im Gegensatz zur Lage des Kniegelenks 4 in Extension in Fig. 1 (a) beträgt der Abstand der Mittelpunkte des Markers 11b und des Markers 12b in Fig. 1 (c) in Aufsicht von oben auf den Oberschenkel 2 nach Flexion auf 90° 5 mm, da bei der Flexion des Kniegelenks 4 meist eine Rotation des Kniegelenks 4 stattfindet. Fig. 3 (d) zeigt analog zu Fig. 3 (b) das Bein 1 aus der Seitenansicht, nun jedoch mit dem Kniegelenk 4 in 90° Flexion. Die Messwerte des Abstands der Marker 11a und 11b, sowie 12a und 12b, der zwischen den ventralen Enden der Markerkugeln gemessen wird, haben sich gegenüber Fig. 3(b) ebenfalls verändert. Der Abstand der ventralen Enden der Marker 11a und 11b beträgt in Flexion 44 mm und der Abstand der ventralen Enden der Marker 12a und 12b beträgt 33 mm.

Die Messwerte der Fig. 3 können, wie im Folgenden dargestellt wird, dazu verwendet werden, die Position der Rotationsachse RA des Kniegelenks 4 anhand eines Ellipsenmodells zu bestimmen.

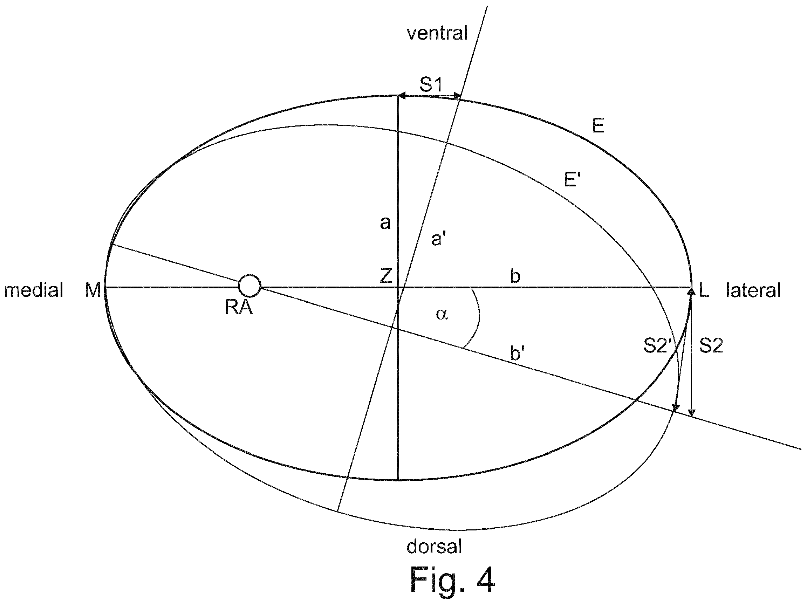

Fig. 4 zeigt die Skizze eines Ellipsenmodells, dass zur Berechnung der Position der Rotationsachse RA des Kniegelenks 4, beispielsweise anhand der zur Fig. 3 beschriebenen Messungen, verwendet werden kann. Die Ellipse E zeigt dabei die Position des Femurkondylen in Extension. Dieses deckt sich in der Extension mit dem Tibiaplateau (Fortsatz des Schienbeins, das zusammen mit dem Femurkondylen das Kniegelenk bildet). Die Ellipse E' zeigt die Position des Femurkondylen in Flexion. Zum Vergleich der Skizze mit der Position des Knies kann Fig. 1 (a) herangezogen werden, in der die mediale und laterale Richtung des Knies ebenfalls gezeigt ist. Der ventrale Scheitel der Ellipse E wäre das obere Ende des Kniegelenks 4, während sich das dorsale Ende der Ellipse E auf der nicht sichtbaren, dem Boden gegenüberliegenden Seite des Kniegelenks 4 befindet. Die kurze Halbachse a der Ellipse E beschreibt die Erstreckung des Kniegelenks 4 in Richtung parallel zur Transversalebene und senkrecht zur Frontalebene des Körpers, also parallel zur Richtung dorsal-ventral. Die lange Halbachse b der Ellipse E beschreibt die Erstreckung des Kniegelenks 4 in Richtung parallel zur Frontalebene und zur Transversalebene des Körpers, also parallel zur Richtung medial-lateral. Die Halbachsen a' und b' der Ellipse E' haben die gleiche Längen wie die Halbachsen a und b der Ellipse E, sind aber um einen Rotationswinkel $\alpha$, welcher der Winkel zwischen der langen Halbachse b des Kniegelenks 4 in Extension und der langen Halbachse b' der Ellipse E' des Kniegelenks 4 in Flexion ist, um die Rotationsachse RA geneigt. Zur Berechnung der kurzen Halbachse a und der langen Halbachse b der Ellipse E wird der ossäre (knöcherne) Umfang U des Kniegelenks verwendet. Der ossäre Umfang U ergibt sich aus der Messung des Knieumfangs abzüglich des Weichteilanteils. Der Weichanteil des Knies ist für einen "schlanken" Patienten mit ca. 10% anzunehmen.

Die Bestimmung der beiden Halbachsen a und b soll im Folgenden dargestellt werden. Ausgangspunkt ist dabei die genäherte Formel für den (ossären) Umfang U der Ellipse E (des Kniegelenks 4):

$$U \cong \pi \cdot \sqrt{2 \cdot (a^2 + b^2)} \qquad (1)$$

Gleichung (1) kann dann durch Quadrieren und Umstellen nach der Summe der quadrierten Längen der Halbachsen aufgelöst werden:

$$(a^2 + b^2) = \left(\frac{U}{\pi}\right)^2 / 2 \qquad (2)$$

Gleichung (2) lässt sich mit dem empirischen Verhältnis

$$\frac{a}{b} \cong \frac{1,5}{2}, \tag{3}$$

welches aus Schätzungen der anatomischen Form des Tibiaplateaus gewonnen wurde, lösen.

In Fig. 4 zeigen S1 und S2' jeweils die relativen ventralen und laterale Positionsänderungen der Femurkondylen, wenn das Kniegelenk von der Extension in die Flexion bewegt wird. Um Berechnungen des Rotationswinkels $\alpha$ des Kniegelenks 4 zu erleichtern, wird für die laterale Positionsänderung der langen Halbachse b die Strecke S2 angenommen. Diese ist ungefähr genauso lang wie die Strecke S2', hat aber den Vorteil, dass sie mit dem Scheitelpunkt L und der Position der Rotationsachse RA ein rechtwinkliges Dreieck bildet (vgl. Fig. 4), wodurch die einfachere Trigonometrie eines rechtwinkligen Dreiecks angewendet werden kann. Der Rotationswinkel $\alpha$ des Kniegelenks 4 kann durch folgende Gleichung näherungsweise bestimmt werden:

$$\alpha = \frac{360°}{\frac{U}{S2'}} \cdot \frac{1,5}{2} \tag{4}$$

Andererseits ist eine Berechnung durch folgende Gleichung möglich:

$$\tan \alpha = \frac{S2}{Strecke\,(L,RA)} \cdot \frac{1,5}{2} \tag{5}$$

Daraus ergibt sich für die *Strecke (L, RA)*:

$$Strecke\,(L,RA) = \frac{S2}{\tan(\alpha)} \tag{6}$$

Umso länger die Strecke (L, RA) zwischen dem Scheitelpunkt L und der Position der Rotationsachse RA ist und solange die Länge der Strecke (L, RA) länger als die Länge der Halbachse b ist, desto medialer liegt die Position der Rotationsachse RA in Bezug auf das Kniegelenkszentrum Z. Im Gegenzug gilt, umso kürzer die Strecke (L, RA) und solange die Strecke (L, RA) kürzer ist als die Strecke der Halbachse b, desto lateraler liegt die Position der Rotationsachse RA in Bezug auf das Kniegelenkszentrum Z.

Eine Beispielberechnung der Position der Rotationsachse RA lässt sich mithilfe der Zahlenwerte der Fig. 3 (b) und Fig. 3 (d) durchführen. Der Umfang des Kniegelenks 4 wird dabei mit $U_{gesamt}$ = 38,5 cm angenommen. Daraus ergibt sich für den ossären Umfang:

$$U_{ossär} = 38,5\ cm - (38,5\ cm \cdot 0,1) = 34,65\ cm$$

Mithilfe der Gleichung (2) ergibt sich für die Länge der Halbachsen $a \cong 47,3$ mm und $b \cong 63$ mm. Dies entspricht einem Verhältnis von 1,5/2, gemäß Gleichung (3). Die relative Positionsänderung der Tibia (des Schienbeins) beim Übergang aus der Extension in eine Flexion mit einem Winkel von 90° beträgt 20 mm. Diese Positionsänderung ergibt sich aus der Differenz der Positionen der ventralen Enden der Marker 12a und 12b beim Übergang von der Extension in die Flexion (53 mm - 33 mm, Fig. 3 (b) und Fig. 3 (d)). Die relative Positionsänderung des Femurs (Oberschenkelknochens) beim Übergang aus der Extension in eine Flexion mit einem Winkel von 90° beträgt 6 mm. Diese Positionsänderung ergibt sich aus der Differenz der Positionen der ventralen Enden der Marker 11a und 11b beim Übergang von der Extension in die Flexion (50 mm - 44 mm, Fig. 3 (b) und Fig. 3 (d)). Die Länge der Strecke S2 kann dann durch die Differenz der relativen Positionsänderung der Tibia und des Femurs beim Übergang von der Extension in die Flexion bestimmt werden und beträgt im gezeigten Beispiel 14 mm (20 mm - 6 mm). Mit der Annahme, dass die beiden Strecken S2 und S2' die gleiche Länge haben, kann mithilfe der Gleichung (4) der Rotationswinkel $\alpha$ bestimmt werden:

$$\alpha = \frac{360°}{\frac{34,65\ cm}{14\ mm}} \cdot \frac{1,5}{2}$$

Daraus ergibt sich ein Rotationswinkel $\alpha = 10{,}9°$. Mithilfe der Gleichung (5) und des Rotationswinkels lässt sich somit die Strecke (L, RA) bestimmen:

$$Strecke\ (L, RA) = \frac{14\ \mathrm{mm}}{\tan(10{,}9°)}$$

Es ergibt sich die Strecke (L, RA)=72,2 mm. Die Position der Rotationsachse RA relativ zum Kniegelenkszentrum Z ergibt sich dann durch die Differenz der Strecke (L, RA) und der langen Halbachse b der Ellipse E und beträgt in diesem Beispiel 9,2 mm (72,2 mm - 63 mm = 9,2 mm). Da dieser Wert positiv ist, liegt die Position der Rotationsachse RA in diesem Beispiel in medialer Richtung um 9,2 mm versetzt zum Kniegelenkszentrums Z.

**Patentansprüche**

1. Verfahren zur Bestimmung des Rotationspunktes eines Kniegelenkes (4) eines Beines (1) einer Person,

   wobei das Kniegelenk (4) einer, mehreren oder sämtlichen der folgenden forcierten Bewegungen, enthaltend Valgusstress, Varusstress, Flexion, Extension und sagittale Translation, ausgesetzt wird, und währenddessen die relativen Positionen des Unterschenkels (3) und des Oberschenkels (2) des Beins (1) bestimmt werden, und aus den bestimmten Positionen der Rotationspunkt des Kniegelenks (4) bestimmt wird.

2. Verfahren nach dem vorhergehenden Anspruch, **dadurch gekennzeichnet, dass** aus den bestimmten Positionen weitere Parameter des Kniegelenks (4) bestimmt werden, wobei die weiteren Parameter des Kniegelenks (4) einen, mehrere oder alle der folgenden Parameter Bandspannung des Innenbandes, Bandspannung des Außenbandes und Bandspannung der Kreuzbänder enthalten

3. Verfahren nach einem der vorhergehenden Ansprüche, **dadurch gekennzeichnet, dass** das Verfahren am gesunden Knie und/oder am kranken Knie und/oder am behandelten Knie durchgeführt wird.

4. Verfahren nach einem der vorhergehenden Ansprüche, **dadurch gekennzeichnet, dass** die forcierte Bewegung durch einen Dritten, durch eine Vorrichtung oder durch eine Bewegung der Person, insbesondere durch den Gang der Person, erzeugt wird.

5. Verfahren nach einem der vorhergehenden Ansprüche, **dadurch gekennzeichnet, dass** die Positionen des Unterschenkels (3) und des Oberschenkels (2) mittels einer oder mehrerer Kameras und/oder mittels eines oder mehrerer am Bein (1) angebrachter Positions- und/oder Bewegungssensoren und/oder mittels eines oder mehrerer am Bein (1) angebrachter Marker (11a, 11b, 12a, 12b), beispielsweise Markerkreuze oder Markerkugeln, erfasst wird.

6. Verfahren nach dem vorhergehenden Anspruch, **dadurch gekennzeichnet, dass** als Positions- und/oder Bewegungssensoren Gyroskope verwendet werden.

7. Verfahren nach einem der beiden vorhergehenden Ansprüche, **dadurch gekennzeichnet, dass** als Position- und/oder Bewegungssensoren mindestens zwei Sensoren verwendet werden, von denen je mindestens einer am Unterschenkel (3) und am Oberschenkel (2) angeordnet ist.

8. Verfahren zur Herstellung einer Kniegelenksendoprothese oder einer kniegelenksübergreifenden Orthese, **dadurch gekennzeichnet, dass** Parameter des Kniegelenks (4) einer Person nach einem der vorhergehenden Verfahren bestimmt werden und die Kniegelenksendoprothese oder Orthese auf der Grundlage der bestimmten Parameter des Kniegelenks (4) hergestellt und/oder implantiert bzw. die Kniegelenksorthese angepasst wird.

9. Verfahren nach dem vorhergehenden Anspruch, **dadurch gekennzeichnet, dass** Parameter des Kniegelenks (4) einer Vielzahl von Personen bestimmt werden und eine Kniegelenksendoprothese oder einer kniegelenksübergreifenden Orthese auf der Grundlage der bestimmten Parameter, insbesondere auf aus den bestimmten Parametern abgeleiteten statistischen Parametern, hergestellt wird.

10. Vorrichtung zur Durchführung eines Verfahrens nach einem der Ansprüche 1 bis 7,

mit einer Positionsbestimmungsvorrichtung zur Bestimmung der relativen Positionen des Unterschenkels (3) und des Oberschenkels (2) des Beins, wenn das Kniegelenk (4) einer, mehreren oder sämtlichen der folgenden forcierten Bewegungen, enthaltend Valgusstress, Varusstress, Flexion, Extension und sagittale Translation, ausgesetzt wird,

**gekennzeichnet, durch**

eine Parameterbestimmungsvorrichtung zur Bestimmung des Rotationspunktes des Kniegelenks (4) aus den durch die Positionsbestimmungsvorrichtung bestimmten Parameter.

11. Vorrichtung nach dem vorhergehenden Anspruch, **dadurch gekennzeichnet, dass** die Parameterbestimmungsvorrichtung weiterhin eingerichtet ist zur zur Bestimmung weiterer Parameter des Kniegelenks (4) aus den durch die Positionsbestimmungsvorrichtung bestimmten Parametern, wobei die weiteren Parameter des Kniegelenks (4) einen, mehrere oder alle der folgenden Parameter Bandspannung des Innenbandes, Bandspannung des Außenbandes und Bandspannung der Kreuzbänder des Kniegelenks (4) enthalten.

## Claims

1. A method for determining the rotation point of a knee joint (4) of a person's leg (1),

   wherein the knee joint (4) is subjected to one, several or all of the following forced movements, including valgus stress, varus stress, flexion, extension and sagittal translation, and
   during which the relative positions of the lower part (3) and the upper part (2) of the leg (1) are determined,
   and the rotation point of the knee joint (4) is determined from the determined positions.

2. The method according to the preceding claim, **characterized in that** further parameters of the knee joint (4) are determined from the determined positions, wherein the further parameters of the knee joint (4) include one, several or all of the following parameters: ligament tension of the inner ligament, ligament tension of the outer ligament and ligament tension of the cruciate ligaments.

3. The method according to any one of the preceding claims, **characterized in that** the method is carried out on a healthy knee and/or on a diseased knee and/or on a treated knee.

4. The method according to any one of the preceding claims, **characterized in that** the forced movement is generated by a third party, by a device or by a movement of the person, in particular by the person's gait.

5. The method according to any one of the preceding claims, **characterized in that** the positions of the lower leg (3) and the upper leg (2) are established by means of one or more cameras and/or by means of one or more position and/or movement sensors attached to the leg (1) and/or by means of one or more markers (11a, 11b, 12a, 12b) attached to the leg (1), for example marker crosses or marker balls.

6. The method according to the preceding claim, **characterized in that** gyroscopes are used as positions and/or movement sensors.

7. The method according to one of the two preceding claims, **characterized in that** at least two sensors are used as position and/or movement sensors, at least one of which is arranged on the shank (3) and at least one of which is arranged on the thigh (2).

8. A method for producing a knee joint endoprosthesis or a knee joint-covering orthosis, **characterized in that** parameters of a person's knee joint (4) are determined according to any one of the preceding methods and the knee joint endoprosthesis or orthosis is produced and/or implanted or the knee joint orthosis is adjusted on the basis of the determined parameters of the knee joint (4).

9. The method according to the preceding claim, **characterized in that** parameters of the knee joint (4) of a plurality of persons are determined and
   a knee joint endoprosthesis or a knee joint-covering orthosis is produced on the basis of the determined parameters, in particular on the basis of statistical parameters derived from the determined parameters.

10. A device for performing a method according to any one of claims 1 to 7,

comprising a position-determining device for determining the relative positions of the shank (3) and the thigh (2), when the knee joint (4) is subjected to one, several or all of the following forced movements, including valgus stress, varus stress, flexion, extension and sagittal translation,
**characterized by**
a parameter determining device for determining the rotation point of the knee joint (4) from the parameters determined by the position determining device.

11. The device according to the preceding claim, **characterized in that** the parameter determining device is further configured to determine further parameters of the knee joint (4) from the parameters determined by the position determining device, wherein the further parameters of the knee joint (4) include one, several or all of the following parameters: ligament tension of the inner ligament, ligament tension of the outer ligament and ligament tension of cruciate ligaments of the knee joint (4).

**Revendications**

1. Procédé pour déterminer le point de rotation d'une articulation de genou (4) de la jambe (1) d'une personne,

    dans lequel l'articulation du genou (4) est soumise à un, plusieurs ou la totalités des mouvements forcés suivants, y compris une contrainte de valgus, une contrainte de varus, une flexion, une extension et une translation sagittale, et
    dans le même temps les positions relatives de la région crurale (3) et de la région fémorale (2) de la jambe (1) sont déterminées,
    et le point de rotation de l'articulation de genou (4) est déterminé à partir des positions déterminées.

2. Procédé selon la revendication précédente, **caractérisé en ce que** des paramètres supplémentaires de l'articulation de genou (4) sont déterminés à partir des positions déterminées, dans lequel les paramètres de l'articulation de genou (4) incluent un, plusieurs ou la totalité des paramètres suivants : tension ligamentaire du ligament interne, tension ligamentaire du ligament externe et tension ligamentaire des ligaments croisés.

3. Procédé selon l'une des revendications précédentes, **caractérisé en ce que** le procédé est réalisé sur un genou sain et/ou sur un genou malade et/ou sur un genou traité.

4. Procédé selon l'une quelconque des revendications précédentes, **caractérisé en ce que** le mouvement forcé est généré par un tiers, par un dispositif ou par un mouvement de la personne, notamment par la démarche de la personne.

5. Procédé selon l'une des revendications précédentes, **caractérisé en ce que** les positions de la région crurale (3) et de la région fémorale (2) sont détectées au moyen d'une ou plusieurs caméras et/ou au moyen d'un ou plusieurs capteurs de position et/ou de mouvements fixés à la jambe (1) et/ou au moyen d'un ou plusieurs marqueurs (11a, 11b, 12a, 12b) fixés à la jambe (1), par exemple des croix de marquage ou des billes de marquage.

6. Procédé selon la revendication précédente, **caractérisé en ce que** des gyroscopes sont utilisés en tant que capteurs de position et/ou de mouvement.

7. Procédé selon l'une des deux revendications précédentes, **caractérisé en ce que** sont utilisés en tant que capteurs de position et/ou de mouvement au moins deux capteurs, dont au moins un est agencé au niveau de la région crurale (3) et au niveau de la région fémorale (2).

8. Procédé de fabrication d'une endoprothèse d'articulation de genou ou d'une orthèse d'articulation de genou, **caractérisé en ce que** les paramètres de l'articulation de genou (4) d'une personne sont déterminés selon l'un quelconque des procédés précédents et l'endoprothèse ou l'orthèse d'articulation genou est fabriquée et/ou implantée sur la base des paramètres déterminés de l'articulation de genou (4) ou l'orthèse d'articulation du genou est ajustée.

9. Procédé selon la revendication précédente, **caractérisé en ce que** les paramètres de l'articulation de genou (4) sont déterminés par une pluralité de personnes, et
une endoprothèse d'articulation de genou ou une orthèse d'articulation de genou est réalisée sur la base des paramètres déterminés, en particulier sur la base de paramètres statistiques dérivés des paramètres déterminés.

**10.** Dispositif pour exécuter un procédé selon l'une quelconque des revendications 1 à 7,

avec un dispositif de détermination de position pour déterminer les positions relatives de la région crurale (3) et de la région fémorale (2) de la jambe lorsque l'articulation de genou (4) est soumise à un, plusieurs ou la totalités des mouvements forcés suivants, y compris une contrainte de valgus, une contrainte de varus, une flexion, une extension et une translation sagittale,
**caractérisé par**
un dispositif de détermination de paramètres pour déterminer le point de rotation de l'articulation de genou (4) à partir des paramètres déterminés par le dispositif de détermination de position.

**11.** Dispositif selon la revendication précédente, **caractérisé en ce que** le dispositif de détermination de paramètres est en outre configuré pour déterminer des paramètres supplémentaire de l'articulation de genou (4) à partir des paramètres déterminés par le dispositif de détermination de position, dans lequel les paramètres supplémentaires de l'articulation du genou (4) sont un, plusieurs ou la totalité des paramètres suivants : tension ligamentaire du ligament interne, tension ligamentaire du ligament externe et tension ligamentaire des ligaments croisés (4).

Fig. 1

Fig. 2

(a)

3, 12b, 14, 12a, 4, 11a, 13, 11b, 2, 1, 20mm

(b)

53mm, 3, 12a, 14, 12b, 4, 11b, 50mm, 11a, 13

(c)

5mm, 14, 12a, 4, 11a, 13, 12b, 2, 1, 11b, 7

(d)

4, 12b, 33 mm, 12a, 11b, 44 mm, 14, 3, 2, 1, 11a, 7, 13

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• WO 2018213749 A1

**In der Beschreibung aufgeführte Nicht-Patentliteratur**

• Stabilitätsprüfung des Kniegelenkes. **BARIE, ALEXANDER.** Themenheft: Diagnostik des Kniebandapparates. 2016